# EUROPÄISCHE PATENTSCHRIFT

(11) **EP 0 491 971 B1**
(45) Veröffentlichungstag und Bekanntmachung des Hinweises auf die Patenterteilung: **07.07.1993**
(21) Anmeldenummer: 90125048.0
(22) Anmeldetag: 20.12.1990
(51) Int. Cl.: A61M 27/00

(54) **Anordnung zur Überwachung von mittels Drainageschläuchen abgeleiteten Körperflüssigkeiten**
Device to monitor body fluids flowing in a drainage tube
Dispositif pour contrôler des liquides corporels s'écoulant par un tube de drainage

(43) Veröffentlichungstag der Anmeldung: 01.07.1992
(73) Patentinhaber: Bleeker, Thomas, Dipl. Ing., D-81241 München (DE)
(72) Erfinder: Bleeker, Thomas, Dipl. Ing., D-81241 München (DE)
(74) Vertreter: Schwepfinger, Karl-Heinz, Dipl.-Ing.

(56) Entgegenhaltungen:
- EP-A- 0 346 548
- WO-A-81/01655
- GB-A- 2 217 736
- US-A- 4 496 346
- IBM TECHNICAL DISCLOSURE BULLETIN Bd. 17, Nr. 5, Oktober 1974, Seiten 1282 - 1283; KROSNER ET AL.: 'FLUID PATH IN-LINE DETECTION FLOW, PRESSURE, CONDUCTIVITY AND/OR OPACITY '

## Beschreibung

Die Erfindung bezieht sich auf eine Anordnung zur Überwachung von mittels Drainageschläuchen abgeleiteten Körperflüssigkeiten gemäß dem Oberbegriff des Anspruchs 1.

Nach chirurgischen Eingriffen werden häufig im Bereich der Operationswunde Drainagen gelegt, mit deren Hilfe Körperflüssigkeiten, die sich als Operationsfolge bilden, nach außen abgeleitet werden können. Durch solche Drainageschläuche fließt auch Blut nach außen ab, wenn es im Operationsbereich zu postoperativen Blutungen kommt. Es ist zwingend notwendig, das Auftreten solcher Blutungen möglichst frühzeitig zu erkennen, damit verhindert wird, daß der Patient verblutet. Bisher ist es üblich, die durch den jeweiligen Drainageschlauch austretende Körperflüssigkeit optisch auf das Vorhandensein von Blut zu überwachen. Sollte eine Nachblutung auftreten, verfärbt sich die austretende Körperflüssigkeit dunkel, was durch den Drainageschlauch sofort zu erkennen ist, der zu diesem Zweck aus durchsichtigem Material hergestellt ist. Die ständige Überwachung einer größeren Anzahl von frisch operierten Patienten in kurzen Zeitabständen erfordert jedoch einen hohen Personalaufwand, so daß insbesondere während der Nachtstunden nicht immer gewährleistet werden kann, daß die Überwachungsabstände ausreichend kurz gehalten sind, um gegebenenfalls schwerwiegende Folgen postoperativer Blutungen für den Patienten gänzlich auszuschließen.

Eine Anordnung dieser Art ist aus "IBM Technical Disclosure Bulletin", Bd.17, Nr. 5, Oktober 74, Seiten 1282 bis 1283 bekannt. Mit Hilfe dieser bekannten Anordnung kann eine Dialysat-Leitung daraufhin überwacht werden, ob die in ihr fließende Flüssigkeit lichtdurchlässig oder lichtundurchlässig ist. Der lichtundurchlässige Zustand, der durch die Anwesenheit von Blut in der Dialysat-Leitung hervorgerufen wird, zeigt einen für den Patienten gefährlichen Zustand an und löst aufgrund der Wirkung der bekannten Überwachungsanordnung einen Alarm aus. Diese Alarmauslösung erfolgt dabei ohne Rücksicht auf die Dauer des lichtundurchlässigen Zustandes der Flüssigkeit in der Dialysat-Leitung. Es gibt jedoch in der Praxis Fälle, in denen ein kurzzeitiges Auftreten von Blut in der von der Körperflüssigkeit durchflossenen Leitung noch nicht gleichbedeutend mit einer Gefährdung des Patienten ist; bei Anwendung der bekannten Anordnung würden aber bereits einzelne Blutstropfen in der Körperflüssigkeit zur Auslösung des Alarms führen.

Der Erfindung liegt die Aufgabe zugrunde, eine Anordnung der eingangs geschilderten Art zu schaffen, die es ermöglicht, ohne großen Personalaufwand auch eine größere Anzahl von Patienten jederzeit und zuverlässig hinsichtlich des Auftretens postoperativer Nachblutungen zu überwachen und nur dann einen Alarm auszulösen, wenn eine tatsächliche Patientengefährdung vorliegt.

Zur Lösung dieser Aufgabe ist die erfindungsgemäße Überwachungsanordnung dadurch gekennzeichnet, daß die Auswertungsschaltung eine erste Zeitmeßeinheit enthält, die die Dauer des Vorliegens der Unterschreitung des Sollwerts mißt und das Alarmsignal abgibt, wenn eine vorgegebene Dauer überschritten wird, und daß die Auswertungsschaltung eine zweite Zeitmeßeinheit enthält, die den Beginn der Zeitmessung durch die erste Zeitmeßeinheit um eine vorgegebene Wartezeit verzögert, die wesentlich kürzer als die vorgegebene Dauer ist.

Bei Anwendung der erfindungsgemäßen Anordnung erfolgt eine Alarmauslösung nur dann, wenn eine Nachblutung auftritt, die länger als eine vorgegebene Zeitdauer anhält. Damit kurzzeitige Nachblutungen, beispielsweise einzelne Blutstropfen in der überwachten Körperflüssigkeit, nicht bereits die die Nachblutungsdauer bestimmende Zeitmeßeinrichtung auslösen, beginnt die Zeitmessung aufgrund der Wirkung der zweiten Zeitmeßeinheit erst dann, wenn nach Beginn der Nachblutung eine vorgegebene Wartezeit abgelaufen ist. Ist die Dauer der Nachblutung kürzer als diese Wartezeit, dann wird die erste Zeitmeßeinheit gar nicht aktiviert.

Vorteilhafte Weiterbildungen der Erfindung sind in den Unteransprüchen gekennzeichnet.

Die Erfindung wird nun anhand der Zeichnung beispielshalber erläutert. Es zeigen:
- Fig. 1: eine schematische Darstellung der erfindungsgemäßen Anordnung und
- Fig. 2: ein Ausführungsbeispiel einer in der erfindungsgemäßen Anordnung verwendbaren Auswertungsschaltung.

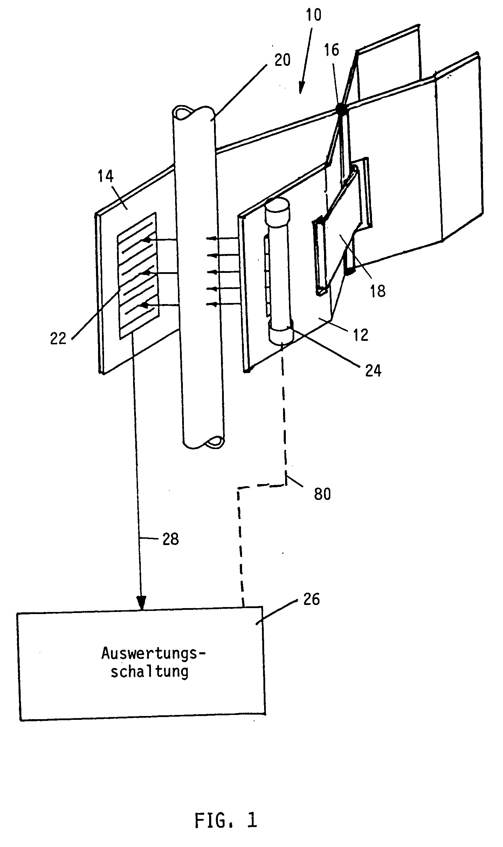

Die in Fig. 1 dargestellte Anordnung enthält eine Klammer 10 mit zwei Klemmarmen 12 und 14, die nach Art einer Wäscheklammer an einem Schwenkpunkt 16 miteinander verbunden sind. Durch Drücken auf die in Fig. 1 rechts des Schwenkpunkts 16 liegenden enden der Klemmarne 12 und 14 können die links des Schwenkpunkts 16 liegenden Enden auseinandergespreizt werden. Mittels einer Feder 18 sind die Klemmarne 12 und 14 so vorgespannt, daß ihre links des Schwenkpunkts liegenden Enden gegeneinandergedrückt werden. Die Klammer 10 ist im geöffneten Zustand, also mit auseinandergeschwenkten linken Enden der Klemmarne 12 und 14 dargestellt.

Zwischen den beiden Klemmarmen 12 und 14 ist ein Stück eines Drainageschlauchs 20 dargestellt, mit dessen Hilfe Körperflüssigkeiten aus einem Patienten abgeleitet werden.

Die Klammer 10 kann an dem Drainageschlauch 20 so festgeklemmt werden, daß die Innenseiten der Klemmarme 12 und 14 beiderseits des Drainageschlauchs 20 zu liegen kommen. Wie in Fig. 1 zu erkennen ist, ist an der mit dem Drainageschlauch 20 in Kontakt kommenden Innenseite des Klemmarms 14 ein schematisch dargestellter Photowiderstand 22 angebracht, während am entsprechenden Teil des anderen Klemmarms 20 eine Lichtquelle 24 in Form mehrerer, in einer Linie angeordneter Leuchtdioden befestigt ist. Die lichtemittierenden Flächen der Leuchtdiode sind dabei zum Drainageschlauch 20 hin gerichtet. Im angeklemmten Zustand der Klammer 10 liegt der Drainageschlauch 20 im Weg des Lichts von den die Lichtquelle 24 bildenden Leuchtdioden zum Photowiderstand 22.

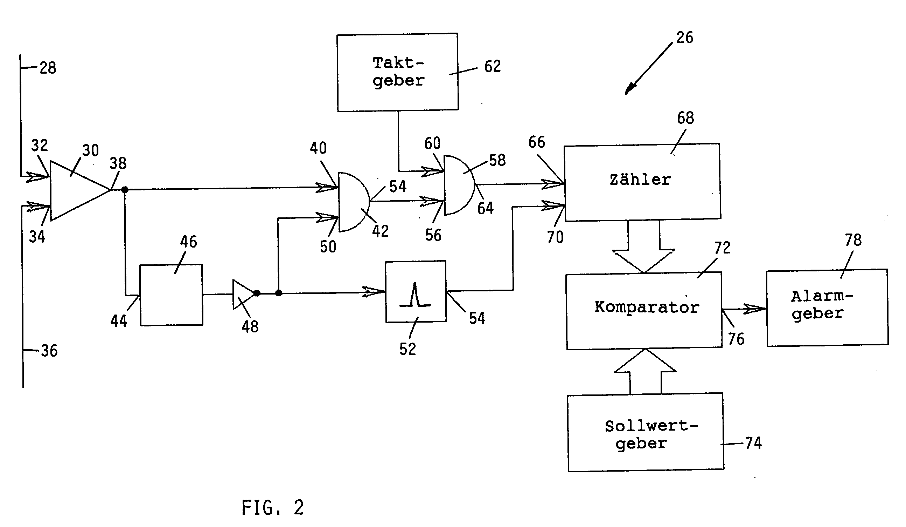

Das Ausgangssignal des Photowiderstandes 22 wird einer in Fig. 1 schematisch dargestellten Auswertungsschaltung 26 über eine Leitung 28 zugeführt. Ein Ausführungsbeispiel dieser Auswertungsschaltung ist in Fig. 2 dargestellt.

Nach Fig. 2 enthält die Auswertungsschaltung 26 einen Komparator 30, der an einem Eingang 32 über die Leitung 28 das Ausgangssignal des Photowiderstandes 22 empfängt. An einem anderen Eingang 34 wird dem Komparator 30 über eine Leitung 36 ein Signal zugeführt, das einen einer vorgegebenen Lichtdurchlässigkeit der Körperflüssigkeit im Drainageschlauch 20 repräsentierenden Sollwert darstellt. Der Ausgang 38 des Komparators ist mit einem Eingang 40 einer UND-Schaltung 42 verbunden. Ferner ist er mit dem Eingang 44 einer monostabilen Kippschaltung (Monoflop) 46 verbunden. Diese monostabile Kippschaltung ist so ausgebildet, daß sie ihr Ausgangssignal für eine vorgegebene Zeitdauer vom Signalwert "0" auf den Signalwert "1" umschaltet, sobald sie am Eingang 44 ein Signal mit dem Signalwert "1" empfängt.

Das Ausgangssignal der monostabilen Kippschaltung 46 gelangt über einen Negator 48 zu einem zweiten Eingang 50 der UND-Schaltung 42 sowie zu einem Rückstellschalter 52, der an seinem Ausgang 54 immer dann ein Rückstellsignal abgibt, wenn das Signal an seinem Eingang vom Wert "0" auf den Wert "1" übergeht.

Der Ausgang 54 der UND-Schaltung 42 ist mit einem Eingang 56 einer weiteren UND-Schaltung 58 verbunden, deren anderer Eingang 60 Taktsignale aus einem Taktgeber 62 empfängt. Der Ausgang 64 der UND-Schaltung 58 ist mit dem Zähleingang 66 eines Zählers 68 verbunden, der mit jedem Empfang eines Taktimpulses aus dem Taktgeber 62 seinen Zählerstand fortschaltet. Der Zähler 68 weist auch einen Rückstelleingang 70 auf, der mit dem Ausgang 54 des Rückstellschalters 52 verbunden ist. Mit dem Empfang eines Rückstellsignals aus dem Rückstellschalter 52 wird der Zähler 68 jeweils auf einen definierten Anfangszustand, beispielsweise den Stand "0", zurückgestellt.

Der Stand des Zählers 68 kann mittels eines weiteren Komparators 72 mit einem Sollwert verglichen werden, der dem Komparator 72 von einem Sollwertgeber 74 zugeführt wird. Mit dem Ausgang 76 des Komparators ist ein Alarmgeber 78 verbunden, der immer dann einen Alarm erzeugt, wenn ihm vom Komparator 72 ein Auslösesignal zugeführt wird.

Die oben beschriebene Anordnung verhält sich im praktischen Einsatz wie folgt:

Zur Vorbereitung der Patientenüberwachung wird die Klammer 10 an einem Drainageschlauch 20 festgeklemmt, der zur Ableitung von Körperflüssigkeiten im Bereich einer am Patienten durchgeführten Operation angebracht ist. Die Klammer 10 ist dabei am sensorseitigen Ende so ausgebildet, daß bei ihrer Anbringung ausschließlich das von der Lichtquelle 24 ausgehende Licht durch den Schlauch hindurch auf den Photowiderstand 22 treffen kann.

Im Anschluß an das Anbringen der Klammer 10 wird durch Einschaltung der Stromversorgung der Anordnung der von Leuchtdioden gebildeten Lichtquelle 24 über die in Fig. 1 gestrichelt angegebene Leitung 80 Strom zugeführt, der die Abstrahlung von Licht durch die Lichtquelle 24 bewirkt. Falls sich im Drainageschlauch 20 eine lichtdurchlässige Körperflüssigkeit befindet, gelangt das Licht durch den aus durchsichtigem Kunststoff hergestellten Drainageschlauch 20 mit nur geringer Abschwächung auf den Photowiderstand 22, so daß sich dieser auf einen bestimmten Widerstandswert einstellt. Das über die Leitung 28 zur Auswertungsschaltung 26 gelangende Signal kann entweder der Spannungsabfall am Photowiderstand 22 oder der durch diesen fließende Strom sein. Für die nachfolgende Beschreibung sei angenommen, daß als Signal der Spannungsabfall in der Auswertungsschaltung 26 ausgenutzt wird. Dieser Spannungsabfall wird dem Eingang 32 des Komparators 30 zugeführt. Am anderen Eingang 34 des Komparators 30 liegt ebenfalls ein Spannungswert, der, wie oben bereits angegeben wurde, als Schwellenwert dient, mit dessen Hilfe unterschieden werden kann, ob die Flüssigkeit im Drainageschlauch 20 lichtdurchlässig oder nicht lichtdurchlässig ist. Der Zustand der Lichtundurchlässigkeit tritt ein, wenn Blut durch den Drainageschlauch 20 fließt.

Es wird nun angenommen, daß kein Blut durch den Drainageschlauch 20 fließt und daß das von der Lichtquelle 24 abgestrahlte Licht nahezu ungedämpft auf den Photowiderstand 22 fallen kann. Der Spannungswert am Eingang 34 des Komparators 30, der als Schwellenwert dient, ist so eingestellt, daß er höher als der sich in diesem Fall ergebende Spannungsabfall am Photowiderstand 22 ist. Der Komparator 30 gibt in diesem Fall an seinem Ausgang 38 ein Signal mit dem Wert "0" ab. Dieses Signal gelangt zum Eingang 40 der UND-Schaltung 42, die dadurch an ihrem Ausgang 54 ebenfalls ein Signal mit dem Wert "0" abgibt. Das Ausgangssignal des Komparators 30 liegt auch am Eingang 44 der monostabilen Kippschaltung 46, die in dem geschilderten Zustand am Ausgang ebenfalls ein Signal mit dem Wert "0" abgibt. Der Negator 48 invertiert dieses Signal, so daß am Eingang 50 der UND-Schaltung 42 ein Signal mit dem Wert "1" anliegt. Das Ausgangssignal der UND-Schaltung 42 mit dem Wert "0" sperrt über den Eingang 56 auch die UND-Schaltung 58, so daß diese die Taktsignale aus dem Taktgeber 62 nicht zum Eingang 66 des Zählers 58 durchläßt. Der Zähler bleibt somit fest auf dem Zählerstand stehen, den er bei der Inbetriebnahme eingenommen hat.

Wenn eine Nachblutung im Körper des Patienten auftritt, füllt sich der Drainageschlauch 20 mit Blut, so daß das Licht, das von der Lichtquelle 24 ausgeht, blockiert wird und nicht mehr auf den Photowiderstand 22 auftrifft. Dies führt zu einer deutlichen Erhöhung des Spannungsabfalls am Photowiderstand 22, so daß die dem Eingang 32 des Komparators 30 zugeführte Spannung größer als die Spannung am Eingang 34 wird. Dadurch gibt der Komparator 30 am Ausgang 38 ein Signal mit dem Wert "1" ab, das zum Eingang 40 der UND-Schaltung 42 gelangt. Gleichzeitig löst das Signal mit dem Wert "1" die monostabile Kippschaltung 46 aus, die dadurch für die Dauer ihrer fest eingestellten Haltezeit in einen Zustand übergeht, in dem sie an ihrem Ausgang ein Signal mit dem Wert "1" abgibt. Dieses Signal wird im Negator 28 negiert, so daß zum Eingang 50 der UND-Schaltung 42 ein Signal mit dem Wert "0" gelangt. Dadurch bleibt die UND-Schaltung 42 weiterhin gesperrt, so daß der Zähler 68 über die ebenfalls gesperrte UND-Schaltung 58 keine Taktimpulse aus dem Taktgeber 62 erhält. Nach Ablauf der fest eingestellten Haltezeit, die eine Sekunde betragen kann, geht die monostabile Kippschaltung 46 wieder in ihren Ruhezustand über, so daß sie an ihrem Ausgang wieder ein Signal mit dem Wert "0" abgibt, das über den Negator 48 als Signal "1" an den Eingang 50 der UND-Schaltung 42 gelangt. Ab diesem Zeitpunkt gibt auch diese UND-Schaltung 42 ein Signal mit dem Wert "1" ab, das die UND-Schaltung 58 in den Zustand versetzt, daß sie die Taktimpulse, die ja ebenfalls den Wert "1" haben, zum Zähler 68 durchläßt. Der Zähler 68 beginnt nun mit jedem Taktimpuls seinen Stand zu erhöhen, solange die UND-Schaltung 58 freigegeben ist. Sobald der Zähler 68 einen vorgegebenen Stand erreicht, der dem im Sollwertgeber 64 fest eingestellten Stand entspricht, erkennt der die beiden Werte vergleichende Komparator 72 diesen Zustand der Übereinstimmung und erzeugt als Folge davon an seinem Ausgang 76 ein Alarmsignal, das einen Alarmgeber 78 in Betrieb setzt. Dieser Alarmgeber befindet sich vorzugsweise dort, wo sich das für die Patientenüberwachung zuständige Personal befindet. Bei Auslösung des Alarmgebers kann das Personal somit sofort nach dem Patienten sehen, bei dem eine Blutung aufgetreten ist, und es können dann die erforderlichen Schritte vorgenommen werden.

Der zwischen den Ausgang des Negators 48 und den Rückstelleingang 70 des Zählers 68 eingefügte Rückstellschalter 52 hat den Zweck, den Zähler 68 jeweils dann in einen definierten Anfangszustand zu versetzen, wenn das Signal am Ausgang des Negators 48 den Signalwert "1" annimmt. Um dies zu erreichen, ist der Rückstellschalter vorzugsweise als Differenzierglied ausgebildet, das aus der Signalflanke, die beim Übergang vom Signalwert "0" zum Signalwert "1" einen Impuls erzeugt, der als Rückstellsignal an den Eingang 70 des Zählers 68 gelegt wird. Dadurch wird gewährleistet, daß der Zähler 68 stets mit einem definierten Anfangszustand, beispielsweise vom Zählerstand "0" aus, zu zählen beginnt.

In der beschriebenen Anordnung bilden der Taktgeber 62, der Zähler 68, der Komparator 72 und der Sollwertgeber 74 eine Zeitmeßeinheit, mit deren Hilfe die Dauer des Fließens von Blut durch den Drainageschlauch 20 gemessen werden kann, so daß durch Einstellen des Sollwerts im Sollwertgeber 74 eine vorgegebene Dauer festgelegt werden kann, nach der die Alarmauslösung erfolgen soll.

Die monostabile Kippschaltung 46 hat gemäß der obigen Erläuterung die Wirkung, daß der Zähler 68 nicht sofort zu zählen beginnt, wenn der Komparator 30 durch eine Änderung seines Ausgangssignals anzeigt, daß durch den Drainageschlauch 20 Blut fließt. Diese Maßnahme soll gewährleisten, daß nicht jedesmal dann, wenn eine kurzzeitige, weniger als 1 Sekunde dauernde Verringerung der Lichtdurchlässigkeit der Flüssigkeit im Drainageschlauch 20 stattfindet, ein Zählvorgang ausgelöst wird. Einzelne Bluttropfen in der Körperflüssigkeit, die noch keine gefährliche Nachblutung anzeigen, lösen daher keinen Alarm aus.

Die monostabile Kippschaltung 46 wirkt in der beschriebenen Anordnung somit als eine weitere Zeitmeßeinheit, die bewirkt, daß der Beginn der Zeitmessung durch den Zähler 68 um eine vorgegebene Wartezeit verzögert wird, die wesentlich kürzer als die Dauer ist, nach der ein Alarm ausgelöst werden soll.

Es kann unter Umständen erwünscht sein, einen Alarm auch dann auszulösen, wenn durch den von der Klammer 10 gebildeten Fühler in zeitlichen Abständen voneinander mehrere Blutungen festgestellt worden sind, die zwar jeweils für sich nicht zur Auslösung des Alarmgebers 78 führten, jedoch über einen längeren Zeitraum auch eine Gefährdung des Patienten darstellen können. Beispielsweise kann der Fall eintreten, daß erst nach zwei oder drei mit Abständen voneinander erfolgenden Blutungen die Gesamtdauer dieser Blutungen so lang ist, daß sie, wenn sie zusammenhängend aufgetreten wäre, den Alarmgeber 78 ausgelöst hätte. Diese Auswertung kann einfach dadurch erreicht werden, daß der Zähler 68 nicht bei Beginn jeder Feststellung einer Blutung in den definierten Anfangszustand "0" versetzt wird, sondern immer nur dann, wenn der Alarmgeber 78 ausgelöst worden ist. Der Zähler würde in diesem Fall die einzelnen Zeitperioden, in denen Blutungen aufgetreten sind, summieren, bis der Wert erreicht ist, der unter Mithilfe des Komparators 72 zur Alarmauslösung führt.

## Patentansprüche

1. Anordnung zur Überwachung von mittels Drainageschläuchen (20) abgeleiteten Körperflüssigkeiten mit einem Sensor (10) zum Abtasten der Lichtdurchlässigkeit der Körperflüssigkeit und zum Erzeugen eines von der abgetasteten Lichtdurchlässigkeit abhängigen Ausgangssignals und einer Auswertungsschaltung (26), die das Ausgangssignal des Sensors (10) empfängt und ein Alarmsignal erzeugt, wenn das Ausgangssignal des Sensors (10) das Unterschreiten eines vorgegebenen Sollwerts der Lichtdurchlässigkeit anzeigt, **dadurch gekennzeichnet,** daß die Auswertungsschaltung (26) eine erste Zeitmeßeinheit (62, 68, 72, 74) enthält, die die Dauer des Vorliegens der Unterschreitung des Sollwerts mißt und das Alarmsignal abgibt, wenn eine vorgegebene Dauer überschritten wird, und daß die Auswertungsschaltung (26) eine zweite Zeitmeßeinheit (46) enthält, die den Beginn der Zeitmessung durch die erste Zeitmeßeinheit (62, 68, 72, 74) um eine vorgegebene Wartezeit verzögert, die wesentlich kürzer als die vorgegebene Dauer ist.

2. Anordnung nach Anspruch 1, **dadurch gekennzeichnet,** daß der Ausgang der zweiten Zeitmeßschaltung (46) mit einem Rückstellschalter (52) in Verbindung steht, der die erste Zeitmeßeinheit (62, 68, 72, 74) nach Ablauf der vorgegebenen Wartezeit in einen definierten Anfangszustand stellt.

3. Anordnung nach Anspruch 1 oder 2, **dadurch gekennzeichnet,** daß das Alarmsignal an einen Rückstelleingang der ersten Zeitmeßeinheit (62, 68, 72, 74) anlegbar ist und deren Rückstellung auf einen definierten Anfangszustand auslöst.

4. Anordnung nach einem der vorhergehenden Ansprüche, **dadurch gekennzeichnet,** daß der Sensor (10) eine Lichtquelle (24) und ein lichtempfindliches Element (22) enthält und daß die Lichtquelle (24) und das lichtempfindliche Element (22) an einer Klemmvorrichtung (10) angebracht sind, die so an einem Drainageschlauch (20) festklemmbar ist, daß dieser zwischen der Lichtquelle (24) und dem lichtempfindlichen Element (22) liegt.

5. Anordnung nach Anspruch 4, **dadurch gekennzeichnet,** daß die Klemmvorrichtung (10) eine Klammer ist, die zwei Klemmarme (12, 14) aufweist, und daß die Lichtquelle (24) und das lichtempfindliche Element (22) auf der Innenseite der zwei Klemmarme (12, 14) einander gegenüberliegend angebracht sind.

6. Anordnung nach Anspruch 4 oder 5, **dadurch gekennzeichnet,** daß die Lichtquelle (24) von wenigstens einer Leuchtdiode gebildet ist und daß das lichtempfindliche Element (22) ein Photowiderstand ist.

7. Anordnung nach einem der Ansprüche 4 bis 6, **dadurch gekennzeichnet,** daß die Lichtquelle (24) von mehreren in einer Linie liegenden Leuchtdioden gebildet ist und daß der Photowiderstand (22) rechteckig ausgebildet ist und in seiner Länge der Länge der von den Leuchtdioden gebildeten Linie entspricht.

## Claims

1. An arrangement for monitoring body fluids, removed by drainage hoses (20), comprising a sensor (10) responsive to the transparency to light of the body fluid and for producing an output signal as a function of the sensed transparency to light and a processing circuit (26), which receives the output signal of the sensor (10) and produces an alarm signal, if the output signal of the sensor (10) indicates that a predetermined target value for the transparency to light has been gone below, characterized in that the processing circuit (26) comprises a first time measuring unit (62, 68, 72, 74), which measures the time below the target value and provides the alarm signal, when a predetermined period of time has been exceeded and in that the processing circuit (26) comprises a second time measuring unit (46), which delays the beginning of time measurement by the first time measuring unit (62, 68, 72, 74) by a predetermined wait time, which is substantially shorter than the said predetermined period of time.

2. The arrangement as claimed in claim 1, characterized in that the output of the second time measuring circuit (46) is connected with a reset switch (52), which sets the first measuring unit (62, 68, 72, 74) after the end of the predetermined wait time to a predefined initial condition.

3. The arrangement as claimed in claim 1 or in claim 2, characterized in that the alarm signal is able to be fed to a reset input terminal of the first time measuring unit (62, 68, 72, 74) and causes the reset thereof to a predefined initial condition.

4. The arrangement as claimed in any one of the preceding claims, characterized in that the sensor (10) comprises a source of light (24) and a light-sensitive element (22) and in that the source of light (24) and the light-sensor element (22) are arranged on a gripping device (10) which is able to be so grippingly engaged with a drain hose (20) that the same is between the source of light (24) and the light-sensitive element (22).

5. The arrangement as claimed in claim 4, characterized in that the gripping device (10) is a clamp, which has two clamping arms (12, 14) and in that the source of light (24) and the light-sensitive element (22) are arranged on the inner side of the two clamping arms (12, 14) opposite to one another.

6. The arrangement as claimed in claim 4 or in claim 5, characterized in that the source of light (24) is constituted by at least one light emitting diode and in that the light-sensitive element (22) is a photoresistor.

7. The arrangement as claimed in any one of the claims 4 through 6, characterized in that the source of light (24) is constituted by a plurality of light emitting diodes disposed in a line and the source of light (24) is designed to be rectangular and has a length equal to the line constituted by the light emitting diodes.

## Revendications

1. Dispositif pour la surveillance de liquides physiologiques évacués au moyen de tuyaux de drainage (20), comportant une cellule sensible (10) pour détecter la transparence du liquide physiologique et pour générer un signal de sortie fonction de la transparence détectée, ainsi qu'un circuit d'analyse (26) qui reçoit le signal de sortie de la cellule sensible (10) et génère un signal d'alarme si le signal de sortie de la cellule sensible (10) indique qu'une valeur nominale prédéterminée de la transparence n'a pas été atteinte, caractérisé en ce que le circuit d'analyse (26) comporte une première unité chronométrique (62, 68, 72, 74) qui mesure la durée pendant laquelle la valeur nominale n'a pas été atteinte et qui émet le signal d'alarme si une durée prédéterminée est dépassée, et en ce que le circuit d'analyse (26) comporte une deuxième unité chronométrique (46) qui retarde le début de la mesure du temps effectuée par la première mesure chronométrique (62, 68, 72, 74) d'un délai prédéterminé qui est sensiblement plus court que la durée prédéterminée.

2. Dispositif selon la revendication 1, caractérisé en ce que la sortie de la deuxième unité chronométrique (46) est en liaison avec une commande de remise à zéro (52) qui met l'unité chronométrique (62, 68, 72, 74) dans un état initial défini après l'écoulement du délai d'attente prédéterminé.

3. Dispositif selon la revendication 1 ou 2, caractérisé en ce que le signal d'alarme est applicable à une entrée de remise à zéro de la première unité chronométrique (62, 68, 72, 74) dont la remise à zéro déclenche un état initial défini.

4. Dispositif selon l'une des revendications précédentes, caractérisé en ce que la cellule sensible (10) comporte une source lumineuse (24) et un élément photorécepteur (22), et en ce que la source lumineuse (24) et l'élément photorécepteur (22) sont disposés sur un dispositif de serrage (10) qui peut être fixé sur un tuyau de drainage (20) de telle sorte que ce dernier se situe entre la source lumineuse (24) et l'élément photosensible (22).

5. Dispositif selon la revendication 4 , caractérisé en ce que le dispositif de serrage (10) est une pince qui présente deux bras de serrage (12, 14), et en ce que la source lumineuse (24) et l'élément photosensible (22) sont fixés en face l'une de l'autre sur la face intérieure des deux bras (12, 14) de la pince.

6. Dispositif selon la revendication 4 ou 5, caractérisé en ce que la source lumineuse (24) est constituée d'au moins une diode électroluminescente et en ce que l'élément photosensible (22) est une cellule photoconductrice.

7. Dispositif selon l'une des revendications 4 à 6, caractérisé en ce que la source lumineuse (24) est constituée de plusieurs diodes électroluminescentes situées en une ligne, et en ce que la cellule photoconductrice (22) est de forme rectangulaire et a sa longueur qui correspond à la longueur de la ligne formée par les diodes électroluminescentes.
